(19) Europäisches Patentamt
European Patent Office
Office européen des brevets

(11) Veröffentlichungsnummer: **0 113 028**
**B1**

# (12) EUROPÄISCHE PATENTSCHRIFT

(45) Veröffentlichungstag der Patentschrift: **30.04.86**

(51) Int. Cl.⁴: **C 07 C 127/00,** A 01 N 47/32, C 07 C 87/451

(21) Anmeldenummer: **83111744.5**

(22) Anmeldetag: **24.11.83**

(54) **Substituierte Benzylcycloalkenylharnstoff-Derivate.**

(30) Priorität: **03.12.82 JP 211241/82**

(43) Veröffentlichungstag der Anmeldung: **11.07.84 Patentblatt 84/28**

(45) Bekanntmachung des Hinweises auf die Patenterteilung: **30.04.86 Patentblatt 86/18**

(84) Benannte Vertragsstaaten: **AT BE CH DE FR GB IT LI NL**

(56) Entgegenhaltungen:
**EP - A - 0 000 376**
**US - A - 3 701 807**

**Die Akte enthält technische Angaben, die nach dem Eingang der Anmeldung eingereicht wurden und die nicht in dieser Patentschrift enthalten sind.**

(73) Patentinhaber: **NIHON TOKUSHU NOYAKU SEIZO K.K., No.4, 2-Chome, Nihonbashi Honcho, Chuo-ku Tokyo 103 (JP)**

(72) Erfinder: **Yamada, Yasuo, 5-15-9, Nishihirayama, Hino-shi Tokyo (JP)**
Erfinder: **Saito, Junichi, 3-7-12, Osawa, Mitaka-shi, Tokyo (JP)**
Erfinder: **Tamura, Tatsuo, 1-7-30, Hanenaka Hamura-machi, Nishitama-gun Tokyo (JP)**
Erfinder: **Sakawa, Shinji, 1-14-13, Tamadaira, Hino-shi Tokyo (JP)**

(74) Vertreter: **Schumacher, Günter, Dr. et al, c/o Bayer AG Konzernverwaltung RP Patentabteilung, D-5090 Leverkusen 1 Bayerwerk (DE)**

**EP 0 113 028 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue substituierte Benzylcycloalkenylharnstoff-Derivate, deren Vorstufen, Verfahren zur Herstellung dieser Derivate und dieser Vorstufen sowie Fungizide zum Einsatz in Landwirtschaft und Gartenbau.

Es ist bereits bekannt, daß bestimmte N-(1-Cycloalken-1-yl)harnstoffe bzw. -thioharnstoffe wie beispielsweise N-Benzyl-N-cyclohexen-1 -yl-N'-phenylharnstoff und N-Cyclohexen-1 -yl-N-methyl-N-phenylharnstoff herbizide bzw. fungizide Eigenschaften besitzen (vgl. die US-PSen 3701 807 und 3761 241). Die Herstellung anderer substituierter N-Cycloalkyl-N-benzyl-N'-phenylharnstoff-Derivate und deren Verwendung als Fungizide sind bekannt (vgl. die DE-PS 2732 257).

Weiterhin sind N-Cycloalkyl-N-benzylharnstoff-Derivate mit fungizider Wirkung bekannt (vgl. EP-A 0 000 376).

Neue substituierte Benzylcycloalkenylharnstoff-Derivate der nachstehenden Formel (I)

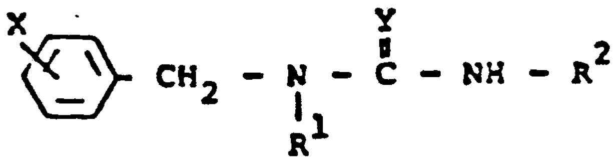

(I)

in der

X ein Halogen-Atom oder eine niedere Alkyl-Gruppe mit 1 bis 6 Kohlenstoff-Atomen bezeichnet,

Y ein Sauerstoff-Atom oder ein Schwefel-Atom bezeichnet,

$R^1$ eine-Cycloalkenyl-Gruppe mit 5 bis 7 Kohlenstoff-Atomen bezeichnet und

$R^2$ eine niedere Alkyl-Gruppe mit 1 bis 6 Kohlenstoff-Atomen, eine Cycloalkyl-Gruppe mit 5 bis 8 Kohlenstoff-Atomen, eine Benzyl-Gruppe oder eine Phenyl-Gruppe bezeichnet, die gegebenenfalls substituiert ist durch 1 bis 5 gleiche oder verschiedene Substituenten, wie Hydroxyl oder eine Alkoxy-Gruppe mit 1 bis 4 Kohlenstoff-Atomen,

wurden nunmehr gefunden.

Weiterhin wurde gefunden, daß neue substituierte Benzylcycloalkenylharnstoff-Derivate der Formel (I)

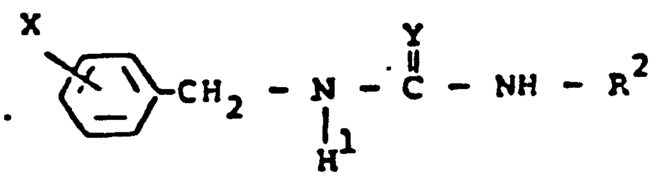

(I)

in der

X ein Halogen-Atom oder eine niedere Alkyl-Gruppe mit 1 bis 6 Kohlenstoff-Atomen bezeichnet,

Y ein Sauerstoff-Atom oder ein Schwefel-Atom bezeichnet,

$R^1$ eine Cycloalkenyl-Gruppe mit 5 bis 7 Kohlenstoff-Atomen bezeichnet und

$R^2$ eine niedere Alkyl-Gruppe mit 1 bis 6 Kohlenstoff-Atomen, eine Cycloalkyl-Gruppe mit 5 bis 8 Kohlenstoff-Atomen, eine Benzyl-Gruppe oder eine Phenyl-Gruppe bezeichnet, die gegebenenfalls substituiert ist durch 1 bis 5 gleiche oder verschiedene Substituenten, wie Hydroxyl oder eine Alkoxy-Gruppe mit 1 bis 4 Kohlenstoff-Atomen,

erhalten werden mittels eines Verfahrens, bei dem

i) ein substituiertes Benzylcycloalkenylamin der Formel (II)

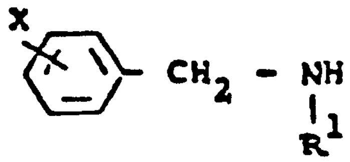

(II)

in der X und $R^1$ die im Vorstehenden angegebenen Bedeutungen haben, umgesetzt wird mit einem Isocyanat der Formel (III)

$$R^2 - N = C = Y \qquad \text{(III)}$$

in der Y und $R^2$ die im Vorstehenden angegebenen Bedeutungen haben, gegebenenfalls in Gegenwart eines Verdünnungsmittels, oder

ii) ein Carbamoylhalogenid der Formel (IV)

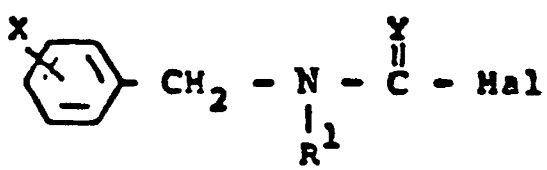

(IV)

in der X, Y und $R^1$ die im Vorstehenden angegebenen Bedeutungen haben und Hal ein Halogen-Atom darstellt, umgesetzt wird mit einem Amin der Formel (V)

$$H_2N - R^2 \qquad \text{(V)},$$

in der $R^2$ die im Vorstehenden angegebene Bedeutung hat, gegebenenfalls in Gegenwart eines Verdünnungsmittels sowie gegebenenfalls in Gegenwart eines säurebindenden Mittels.

Weiterhin wurde gefunden, daß die neuen substituierten Benzylcycloalkenylharnstoff-Derivate der Formel (I) starke fungizide Eigenschaften besitzen. Sie können als Fungizide in Landwirtschaft und Gartenbau eingesetzt werden. Die Substanzen gemäß der vorliegenden Erfindung stellen somit eine Bereicherung der Technik dar.

Die Formel (I) gibt eine allgemeine Definition der substituierten Benzylcycloalkenylharnstoff-Derivate gemäß der vorliegenden Erfindung wieder.

Besonders bevorzugte Verbindungen der Formel (I) sind diejenigen, in denen

X Fluor, Chlor, Brom, Iod, Methyl, Ethyl, n-Propyl, iso-Propyl, n-Butyl, sec-Butyl, iso-Butyl oder tert-Butyl bezeichnet,

Y ein Sauerstoff-Atom oder ein Schwefel-Atom bezeichnet,

$R^1$ 2-Cyclopenten-1 -yl, 3-Cyclopenten-1 -yl, 2-Cyclo-hexen-1 -yl, 3-Cyclohexen-1 -yl, 2-Cyclohepten-1 -yl, 3-Cyclohepten-1 -yl, bezeichnet und

$R^2$ Methyl, Ethyl, n-Propyl, iso-Propyl, n-Butyl, sec-Butyl, iso-Butyl, tert-Butyl, Cyclopentyl, Cyclohexyl, Cycloheptyl, Cyclooctyl, Benzyl, Phenyl, das gegebenenfalls durch 1 oder 2 gleiche oder verschiedene Substituenten substituiert ist, wie Hydroxyl, Methoxy, Ethoxy, n-Propoxy oder iso-Propoxy, bezeichnet.

Wenn beispielsweise N(-2-Cyclohexen-1-yl)-N-(4-chloro-benzyl)-amin und Methylisocyanat als Ausgangssubstanzen verwendet werden, kann der Reaktionsablauf des Verfahrens i) gemäß der vorliegenden Erfindung durch die folgende Gleichung dargestellt werden:

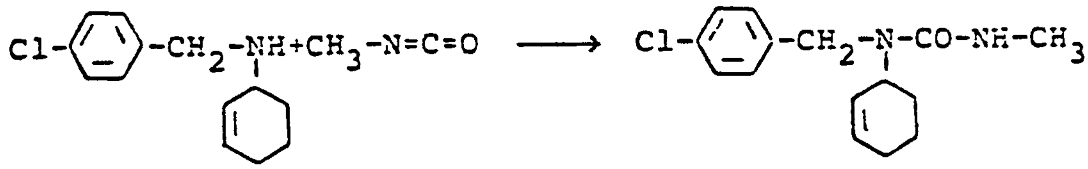

Wenn beispielsweise N-4-Bromobenzyl-N-(2-cyclopenten-1-yl)carbamoylchlorid und Anilin als Ausgangssubstanzen verwendet werden, kann der Reaktionsablauf des Verfahrens ii) gemäß der vorliegenden Erfindung durch die folgende Gleichung dargestellt werden:

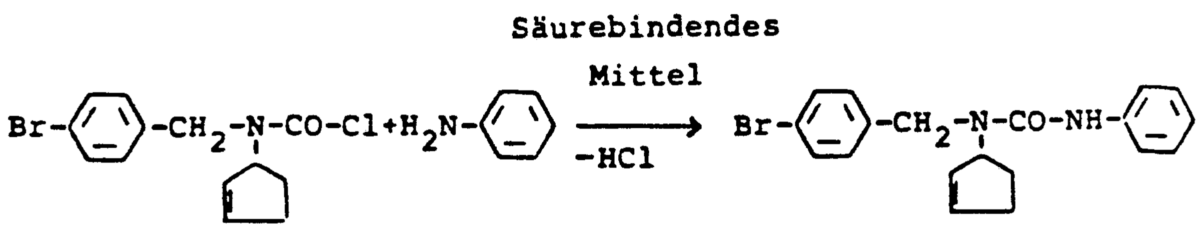


Die substituierten Benzyl-cycloalkenylamine der Formel (II), die als Ausgangsstoffe für das erfindungsgemäße Verfahren i) benötigt werden, sind neu. Die vorliegende Erfindung betrifft ebenfalls diese neuen Zwischenprodukte der Formel (II). Diese Zwischenprodukte können beispielsweise mittels der nachstehenden bekannten Verfahren hergestellt werden;

Verfahren a):

Ein Verfahren zur Herstellung eines substituierten Benzyl-cycloalkenylamins der vorstehenden Formel (II), das gekennzeichnet ist durch Umsetzung eines substituierten Benzylamins der Formel

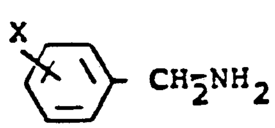 (VI)

in der X die oben angegebene Bedeutung hat, mit einer Verbindung der Formel

$R^1$ - Hal (VII),

in der $R^1$ und Hal die oben angegebenen Bedeutungen haben, oder

Verfahren b):

Ein Verfahren zur Herstellung eines substituierten Benzyl-cycloalkenylamins der vorstehenden Formel (II), das gekennzeichnet ist durch Umsetzung eines substituierten Benzylhalogenids der Formel

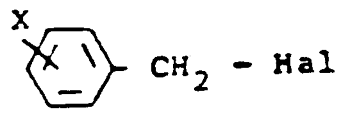

(VIII)

in der X und Hal die oben angegebenen Bedeutungen haben, mit einem Cycloalkenylamin der Formel

$R^1 - NH_2$(IX),

in der $R^1$ die oben angegebene Bedeutung hat.

Die beiden Verfahren a) und b) können gegebenenfalls in Gegenwart eines Verdünnungsmittels und in Gegenwart eines säurebindenden Mittels bei Temperaturen zwischen etwa -20°C und dem Siedepunkt der Reaktionsmischungen, vorzugsweise zwischen 0° C und 100° C, durchgeführt werden.

Spezielle Beispiele für das Ausgangsmaterial der Formel (II) sind

N-4-Chlorobenzyl-2-cyclopenten-1-ylamin,
N-4-Bromobenzyl-2-cyclopenten-1-ylamin,
N-4-Methylbenzyl-2-cyclopenten-1-ylamin,
N-4-Chlorobenzyl-2-cyclohexen-1-ylamin,
N-4-Bromobenzyl-2-cyclohexen-1-ylamin,
N-4-Methylbenzyl-2-cyclohexen-1-ylamin,
N-4-Chlorobenzyl-3-cyclopenten-1-ylamin,
N-4-Bromobenzyl-3-cyclopenten-1-ylamin,
N-4-Chlorobenzyl-2-cyclohepten-1-ylamin.

Die als Ausgangsstoffe für das erfindungsgemäße Verfahren i) benötigten Isocyanate der Formel (III) sind im allgemeinen wohlbekannte Verbindungen.

Spezielle Beispiele für diese Ausgangsstoffe, die Isocyanate der Formel (III), sind

Methylisocyanat, Ethylisocyanat, Propylisocyanat, n-Butyl-isocyanat, Cyclohexylisocyanat Phenylisocyanat, 3-Methoxyphenylisocyanat, Isopropylisocyanat, Ethylisothiocyanat, Phenylisothiocyanat, n-Butylisothiocyanat, Benzylisothiocyanat, 3-Hydroxyphenylisocyanat, 4-Hydroxyphenylisocyanat, 4-Methoxyphenylisocyanat.

Die Formel (IV) gibt eine allgemeine Definition der Carbamoyl-Halogenide, die als Ausgangssubstanzen für das Verfahren ii) gemäß der vorliegenden Erfindung benötigt werden. Sie können mittels bekannter Analogie Verfahren hergestellt werden.

Spezielle Beispiele für diese Ausgangsstoffe. die Carbamoylhalogenide der Formel (IV), sind

N-4-Chlorobenzyl-N-(2-cyclopenten-1-yl)carbamoyl-chlorid,
N-4-Bromobenzyl-N-(2-cyclopenten-1-yl)carbamoyl-chlorid,
N-4-Methylbenzyl-N-(2-cyclopenten-1-yl)carbamoyl-chlorid,
N-4-Chlorobenzyl-N-(2-cyclohexen-1-yl)carbamoyl-chlorid,
N-4-Bromobenzyl-N-(2-cyclohexen-1-yl)carbamoyl-chlorid,
N-4-Methylbenzyl-N-(2-cyclohexen-1-yl)carbamoyl-chlorid,
N-4-Chlorobenzyl-N-(3-cyclopenten-1-yl)carbamoyl-chlorid,
N-4-Bromobenzyl-N-(3-cyclopenten-1-yl)carbamoyl-chlorid,
N-4-Chlorobenzyl-N-(3-cyclohexen-1-yl)carbamoyl-chlorid,
N-4-Methylbenzyl-N-(3-cyclohexen-1-yl)carbamoyl-chlorid,
N-4-Chlorobenzyl-N-(2-cyclohepten-1-yl)carbamoyl-chlorid,

ebenso wie die den obigen Chloriden entsprechenden Bromide sowie die den vorstehenden Carbamoylhalogeniden entsprechenden Thiocarbamoylhalogenide, etwa die Thiocarbamoylchloride bzw. -bromide.

Spezielle Beispiele für die anderen wohlbekannten Ausgangsstoffe, die Amine der Formel (V), die für das erfindungsgemäße Verfahren ii) benötigt werden, sind Methylamin, Ethylamin, Propylamin, n-Butyl-amin, Cyclohexylamin, Anilin, 3-Methoxyanilin, Isopropylamin, m-Aminophenol, p-Aminophenol, 4-Methoxyanilin.

Zu möglichen Verdünnungsmitteln für die erfindungsgemäße Reaktion im Verfahren i) zählen Wasser, aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid und Trichloroethylen, Chlorbenzol etc.; Ether wie Diethylether,

Methylethylether, Di-isopropylether, Dibutylether, Propylenoxid, Dioxan, Tetrahydrofuran etc.; Ketone wie Aceton, Methylethylketon, Methylisopropylketon, Methylisobutylketon etc.; Nitrile wie Acetonitril, Propionitril, Acrylnitril etc.; Ester wie Ethylacetat, Amylacetat etc.; Säureamide wie Dimethylformamid, Dimethylacetamid etc.; Sulfone und Sulfoxide wie Dimethylsulfoxid, Sulfolan etc.; sowie Basen wie Pyridin etc..

Das Verfahren i) gemäß der vorliegenden Erfindung kann bei einer Temperatur innerhalb eines weiten Bereiches durchgeführt werden. Im allgemeinen wird es bei einer Temperatur zwischen etwa -20°C und dem Siedepunkt der Mischung durchgeführt, vorzugsweise zwischen etwa 0°C und etwa 100°C. Obwohl die Reaktion zweckmäßigerweise unter normalem Druck durchgeführt wird, ist es auch möglich, unter erhöhtem oder vermindertem Druck zu arbeiten.

Das Verfahren ii) gemäß der vorliegenden Erfindung kann unter Einsatz eines inerten Lösungsmittels oder Verdünnungsmittels ähnlich den oben erwähnten durchgeführt werden, wodurch das angestrebte Produkt mit hoher Reinheit im hoher Ausbeute erhalten wird.

Weiterhin kann die vorstehende Reaktion ii) in Gegenwart eines säurebindenden Mittels durchgeführt werden. Als solche säurebindende Mittel können erwähnt werden allgemein Hydroxide, Carbonate, Hydrogencarbonate und Alkoholate etc. von Alkalimetallen, sowie tertiäre Amine wie Triethylamin, Diethylanilin und Pyridin etc..

Das Verfahren ii) gemäß der vorliegenden Erfindung kann bei einer Temperatur innerhalb eines weiten Bereichs durchgeführt werden. Im allgemeinen wird es bei einer Temperatur zwischen etwa -20°C und dem Siedepunkt der Mischung durchgeführt, vorzugsweise zwischen etwa 0°C und etwa 100°C. Obwohl die Reaktion zweckmäßigerweise unter normalem Druck durchgeführt wird, ist es auch möglich, unter erhöhtem oder vermindertem Druck zu arbeiten.

Wenn eine Verbindung der Formel (I), in der $R^2$ eine hydroxy-substituierte Phenyl-Gruppe ist, hergestellt werden soll, wird das vorstehende Verfahren i) bevorzugt.

Bei dem Verfahren a) zur Herstellung der neuen Verbindungen der Formel (II) gemäß der vorliegenden Erfindung. wie es im Vorstehenden erläutert wurde, sind spezielle Beispiele für die Ausgangsstoffe, die bekannten substituierten Benzylamine der Formel (VI), 4-Chloro-benzylamin, 4-Bromobenzylamin, 4-Methylbenzylamin etc..

Zu speziellen Beispielen für die anderen Ausgangsstoffe, die Verbindungen der Formel (VII), zählen 3-Chloro-(oder Bromo)-cyclopenten, 3-Chloro(oder Bromo)-cyclohexen, 4-Chloro(oder Bromo)-cyclopenten, 4-Chloro(oder Bromo)-cyclohexen, 3-Chloro(oder Bromo)-cyclohepten etc..

Bei dem Verfahren b) zur Herstellung der neuen Verbindungen der Formel (II) gemäß der vorliegenden Erfindung, wie es im Vorstehenden erläutert wurde, umfassen spezielle Beispiele für die Ausgangsstoffe, die substituierten Benzylhalogenide der Formel (VIII), 4-Chlorobenzylchlorid, 4-Bromobenzylchlorid, 4-Methylbenzylchlorid etc., ebenso die den Chloriden entsprechenden Bromide. Zu speziellen Beispielen für das andere Ausgangsmaterial, die Cycloalkylenamine der Formel (IX), zählen 2-Cyclopenten-1-ylamin, 2-Cyclohexen-1-ylamin, 3-Cyclopenten-1-ylamin, 3-Cyclohexen-1-ylamin, 2-Cyclo-hepten-1-ylamin etc..

Die oben beschriebenen Verfahren a) und b) können unter Einsatz eines inerten Lösungsmittels oder Verdünnungsmittels ähnlich den oben bei den Verfahren i) und ii) erwähnten durchgeführt werden, wodurch das angestrebte Produkt mit hoher Reinheit in hoher Ausbeute erhalten wird.

Weiterhin können die vorstehenden Verfahren a) und b) in Gegenwart eines säurebindenden Mittels ähnlich den oben genannten durchgeführt werden.

Die obigen Verfahren a) und b) können bei einer Temperatur innerhalb eines weiten Bereichs durchgeführt werden. Im allgemeinen wird die Reaktion bei einer Temperatur zwischen etwa -20°C und dem Siedepunkt der Mischung durchgeführt, vorzugsweise zwischen etwa 0°C und etwa 100°C. Obwohl die Reaktion zweckmäßigerweise unter normalem Druck durchgeführt wird, ist es auch möglich, unter erhöhtem oder vermindertem Druck zu arbeiten.

Seitens der Anmelderin wurde gefunden, daß die substituierten Benzylcycloalkenylharnstoff-Derivate der obigen Formel (I). die bisher nirgendwo in der Literatur beschrieben sind, hergestellt werden können, und daß diese Verbindungen eine ausgezeichnete fungizide Wirksamkeit bei der Anwendung in Landwirtschaft und Gartenbau besitzen.

Weiterhin lassen sich die Verbindungen der Formel (I) gemäß der vorliegenden Erfindung leicht herstellen, und ihre Strukturmerkmale bestehen darin, daß die Harnstoff(Thioharnstoff)-Struktur das Gerüst bildet und an das Stickstoff-Atom in 1-Stellung eine substituierte Benzyl-Gruppe und eine Cycloalkenyl-Gruppe mit 5 bis 7 Kohlenstoff-Atomen. insbesondere eine 2-(oder 3-)Cyclo-alken-1-yl-Gruppe und an das Stickstoff-Atom in 3-Stellung ein Rest $R^2$ wie oben definiert gebunden sind. Es wurde nunmehr gefunden, daß diese Verbindungen aufgrund der angegebenen spezifischen Struktur eine land- und gartenwirtschaftliche fungizide Wirkung zu entfalten vermögen, insbesondere eine außerordentlich bemerkenswerte Bekämpfungswirkung gegen Reis-Schalenmehltau (rice sheath blight), der von Pellicularia sasakii verursacht wird.

Weiterhin wurde auch gefunden, daß die Verbindungen der Formel (I) gemäß der vorliegenden Erfindung eine zufriedenstellende Bekämpfungswirkung gegen die Triebfäule und Fußvermorschung von Gemüsepflanzen zu entfalten vermögen, die durch Rhizoctonia solani verursacht wird.

Die aktiven Verbindungen der vorliegenden Erfindung können in wirksamer Weise eingesetzt werden gegen pathogene Pilze, die parasitisch oberirdische Pflanzenteile befallen, pathogene Pilze, die die Pflanzen durch den Boden angreifen und Tracheomykose verursachen, auf Samen lebende pathogene Pilze sowie im Boden lebende pathogene Pilze.

Da diese Verbindungen weiterhin nur eine geringe Toxizität gegen warmblütige Tiere zeigen und eine gute Verträglichkeit mit höheren Pflanzen aufweisen, d.h. gegen Nutzpflanzen keine Phytotoxizität in denjenigen Konzentrationen aufweisen, die üblicherweise angewandt werden, können sie in sehr vorteilhafter Weise gegen Pflanzenkrankheiten, die durch pathogene Pilze verursacht werden, als Fungizide in der Landwirtschaft und im Gartenbau eingesetzt werden.

Die Verbindungen der Formel (I) gemäß der vorliegenden Erfindung können in wirksamer Weise als Fungizide gegen Archimycetes, Phycomycetes, Ascomycetes, Basidiomycetes und Fungi imperfecti sowie gegen verschiedene Pflanzenkrankheiten, die durch andere Bakterien verursacht werden, eingesetzt werden.

Wenn die Verbindungen gemäß der vorliegenden Erfindung als land- oder gartenwirtschaftliche-Fungizide verwendet werden, können sie unmittelbar mit Wasser verdünnt oder aber durch Zusatz landwirtschaftlich unbedenklicher Hilfsstoffe zu verschiedenen Präparaten durch Verfahren verarbeitet werden, die auf dem Gebiet der Herstellung von Agrochemikalien allgemein angewandt werden. Diese Präparate können entweder unmittelbar oder nach dem Verdünnen mit Wasser auf die gewünschte Konzentration zur praktischen Anwendung gelangen.

Die landwirtschaftlich unbedenklichen Hilfsstoffe, auf die hier Bezug genommen wird, umfassen beispielsweise Verdünnungsmittel (Lösungsmittel, Füllstoffe, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe [z.B. n-Hexan, Petrolether, Naphtha, Erdöl-Fraktionen (z.B. Paraffinwachs, Kerosin, Leichtöl, Mittelöl, Schweröl), Benzol, Toluol, Xylole etc.], halogenierte Kohlenwasserstoffe [z.B. Methylenchlorid, Kohlenstofftetrachlorid, Trichloroethylen, Ethylenchlorid, Ethylendibromid, Chlorbenzol, Chloroform etc.], Alkohole [z,B. Methylalkohol, Ethylalkohol, Propylalkohol, Ethylenglycol etc,], Ether [z.B. Diethylether, Ethylenoxid, Dioxan etc.], Alkohol-ether [z.B. Ethylenglycol-monomethylether etc.], Ketone [z.B. Aceton, Isophoron etc.], Ester [z.B. Ethylacetat, Amylacetat etc.], Amide [z.B. Dimethylformamid, Dimethylacetamid etc.] und Sulfoxide [z.B. Dimethylsulfoxid etc.] und dergleichen.

Beispiele für die Füllstoffe oder Träger umfassen anorganische pulverförmige Feststoffe, beispielsweise Löschkalk, Magnesiumkalk, Gips, Calciumcarbonat, Siliciumdioxid, Perlit, Bimsstein, Calcit, Diatomit, amorphes Siliciumdioxid, Aluminiumoxid, Zeolith, Tonminerale (z.B. Pyrophyllit, Talkum, Montmorillonit, Beidellit, Vermikulit, Kaolinit, Glimmer etc.) pflanzliche pulverförmige Feststoffe (wie Getreidepulver, Stärkearten, verarbeitete Stärkearten, Zucker, Glucose, zerkleinerte Stengel von Pflanzen etc.) sowie pulverförmige Feststoffe aus synthetischen Harzen (wie Phenol-Harzen, Harnstoff-Harzen, Vinylchlorid-Harzen etc.) und dergleichen.

Beispiele für oberflächenaktive Mittel umfassen anionische oberflächenaktive Mittel wie Alkylsulfate (z.B. Natriumlaurylsulfat), Arylsulfonsäuren (z,B. Alkylarylsulfonsäure-Salze, Natriumalkylnaphthalinsulfonate etc). Bernsteinsäure-Salze, Salze von Schwefelsäureestern von Polyethylenglycolalkylarylethern, kationische oberflächenaktive Mittel wie Alkylamine (z.B. Laurylamin, Stearyltrimethylammoniumchlorid, Alkyldimethylbenzylammoniumchlorid etc,), Polyoxyethylenalkylamine etc., nicht-ionische oberflächenaktive Mittel wie Polyoxyethylenglycolether (z,B. Polyoxyethylenalkylarylether und deren Kondensate etc.), Polyoxyethylenglycolester (z.B. Polyoxyethylenfettsäureester etc.) und Polyol-Ester (z.B, Polyoxyethylensorbitanmonolaurat etc), ampholytische oberflächenaktive Mittel und so fort.

Beispiele für andere Hilfsstoffe sind Stabilisatoren, Haftmittel [z.B. landwirtschaftliche Seifen, Casein-Kalk. Natriumalginat, Polyvinylalkohol (PVA), Haftmittel auf Vinylacetat-Basis, Acryl-Haftmittel etc.], Aerosol-Treibmittel [/z.B. Trichlorofluoromethan, Dichlorofluoromethan, 1,2,2-Trichloro-1,1.2-trifluoroethan, Chlorbenzol, verflüssigtes Erdgas. niedere Ether etc.], die Verbrennung steuernde Mittel (für Räucherpräparate) [z.B. Nitrite. Zink-Pulver, Dicyandiamid], wirkungsverlängernde Mittel, Dispersions-Stabilisatoren [z.B. Casein, Tragant, Carboxymethylcellulose (CMC) und Polyvinylalkohol (PVA)] und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Beispiele für solche Anwendungsformen sind emulgierbare Konzentrate, Öl-Präparate, benetzbare Pulver, wäßrige Lösungen, Suspensionen, Pulver, Granulate, pulvrige Präparate, Räuchermittel, Tabletten, Aerosole, Pasten, Kapseln etc.. Die land- oder gartenwirtschaftlichen Fungizide gemäß der vorliegenden Erfindung können die vorerwähnten aktiven Wirkstoffe in Mengen von etwa 0,1 bis etwa 95 Gew.-%, vorzugsweise etwa 0,5 bis etwa 90 Gew.-% enthalten.

Für den praktischen Gebrauch können die Gehalte der aktiven Verbindungen in den verschiedenen vorgenannten Mitteln und gebrauchsfertigen Präparaten in angemessener Weise variiert werden, beispielsweise im Bereich von etwa 0,0001 bis etwa 20 Gew.-%, vorzugsweise etwa 0,005 bis etwa 10 Gew.-%.

Der Gehalt des Wirkstoffs kann in geeigneter Weise je nach der Form des Präparats, dem Verfahren, Zweck, der Zeit und dem Ort seiner Anwendung, der Schwere der Pflanzenerkrankung etc. abgeändert werden.

Erforderlichenfalls können die Verbindungen gemäß der vorliegenden Erfindung auch bei gleichzeitiger Anwesenheit anderer Agrochemikalien verwendet werden, beispielsweise von Insektiziden, Fungiziden, Akariziden, Nematiziden, Anti-Virus-Mitteln, Herbiziden, Pflanzen-Wachstumsregulatoren, Lockstoffen [beispielsweise organischen Phosphat-Verbindungen, Carbamat-Verbindungen, Dithio(oder thiol)carbamat-Verbindungen, organischen Chlor-Verbindungen, Dinitro-Verbindungen, organischen Schwefel- oder Metall-

Verbindungen, Antibiotika, substituierten Diphenylether-Verbindungen, Harnstoff-Verbindungen und Triazin-Verbindungen] und/oder Düngemitteln und dergleichen.

Die die im Vorstehenden bezeichneten Wirkstoffe gemäß der vorliegenden Erfindung enthaltenden verschiedenartigen Mittel und gebrauchsfertigen Präparate können mittels eines gebräuchlichen Verfahrens für das Aufbringen zur Anwendung gebracht werden, beispielsweise durch Sprühen [beispielsweise Versprühen von Flüssigkeiten, Vernebeln, Zerstäuben, Stäuben, Streuen von Granulat, Wasser-Oberflächenbehandlung, Gießen etc.] Räuchern, Bodenbehandlung [beispielsweise Einarbeiten in den Boden, Streuen, Bedampfen, Gießen etc.], Oberflächenbehandlung [beispielsweise Beschichten, Bändern, Pulverbeschichten, Bedecken etc.] Imprägnieren und dergleichen. Außerdem können sie auch mittels des sogenannten Ultra-Low-Volume-Verfahrens aufgebracht werden. Nach diesem Verfahren kann die Zusammensetzung sogar 100 % des Wirkstoffs enthalten.

Die Aufwandmengen pro Flächeneinheit betragen etwa 0,03 bis etwa 10 kg/ha, vorzugsweise etwa 0,3 bis etwa 6 kg/ha. In besonders gelagerten Fällen ist es jedoch möglich oder manchmal sogar erforderlich, Aufwandmengen außerhalb des angegebenen Bereichs einzusetzen.

Die vorliegende Erfindung macht fungizide Zusammensetzungen für Landwirtschaft und Gartenbau verfügbar, die die aktiven Verbindungen der vorstehenden Formel (I) als Wirkstoffe sowie Verdünnungsmittel (Lösungsmittel und/oder Füllstoffe und/oder Träger) und/oder oberflächenaktive Mittel und, falls erforderlich, z.B. Stabilisatoren, Haftmittel und Synergisten enthält.

Die vorliegende Erfindung macht weiterhin ein Verfahren zur Bekämpfung von Pflanzenkrankheiten verfügbar, das darin besteht, daß eine Verbindung der allgemeinen Formel (I) allein oder in Form einer Mischung mit einem Verdünnungsmittel (einem Lösungsmittel und/oder einem Füllstoff und/ oder einem Träger) und/oder einem oberflächenaktiven Mittel und, falls erforderlich, z.B. einem Stabilisator, einem Haftmittel, einem synergistischen Mittel, zur Anwendung gebracht wird. Die vorliegende Erfindung wird durch die folgenden Beispiele näher erläutert, ist jedoch nicht auf diese beschränkt.

## A. Herstellungsbeispiele

### Beispiel 1

[Beispiel für die Synthese der Verbindung der Formel (II) gemäß der Erfindung].

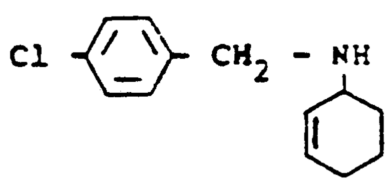

(Verbindung Nr. II-1)

35 g 4-Chlorobenzylamin wurden in 250 ml Toluol gelöst, und unter Rühren und Eiskühlung der Lösung wurde eine Lösung von 20 g 3-Bromocyclohexen in 20 ml Toluol tropfenweise hinzugegeben. Nach der Zugabe wurde die Temperatur allmählich erhöht, und das Rühren wurde bei 50°C etwa 5 h weiter fortgesetzt. Nach dem Abkühlen wurde die Reaktionsmischung unter Absaugen filtriert, und die Toluol-Schicht wurde gründlich mit Wasser gewaschen. Die Toluol-Lösung wurde über wasserfreiem Natriumsulfat getrocknet, das Toluol wurde abdestilliert, und der Rückstand wurde unter vermindertem Druck destilliert, wodurch 15 g des angestrebten Produkts, N-4-Chlorobenzyl-2-cyclohexen-1 -ylamin, erhalten wurden. Sdp. 134-138°C bei 0,73 mbar (0,55 mmHg).

Die Verbindungen der Formel (II) (Zwischenprodukte) gemäß der vorliegenden Erfindung, die nach Verfahrensweisen ähnlich der in Beispiel 1 beschriebenen hergestellt wurden, sind in der Tabelle 1 aufgeführt.

**Tabelle 1**

| Verbindung Nr. | Verbindung der Formel (II) gemäß der Erfindung (Zwischenprodukt) |
|---|---|
| II-2 | N-4-Chlorobenzyl-2-cyclopentenylamin, Sdp. 134-137°C/1,6 mbar (1,2 mmHg) |
| II-3 | N-4-Bromobenzyl-2-cyclopentenylamin, Sdp. 147-152°C/2,0 mbar (1,5 mmHg) |
| II-4 | N-4-Methylbenzyl-2-cyclopentenylamin, Sdp. 120-121°C/2,0 mbar (1,5 mmHg) |
| II-5 | N-4-Methylbenzyl-2-cyclohexenylamin, Sdp. 137-142°C/2,0 mbar (1,5 mmHg) |
| II-6 | N-4-Bromobenzyl-2-cyclohexenylamin Sdp. 157-160°C/1,07 mbar (0,8 mmHg) |
| II-7 | N-4-Chlorobenzyl-3-cyclopentenylamin, |
| II-8 | N-4-Bromobenzyl-3-cyclopentenylamin, |
| II-9 | N-4-Chlorobenzyl-3-cyclohexenylamin, |
| II-10 | N-4-Methylbenzyl-3-cyclohexenylamin, |
| II-11 | N-4-Chlorobenzyl-2-cycloheptenylamin. |

**Beispiel 2**

[Beispiel für die Synthese der Verbindung der Formel (I) gemäß der Erfindung].

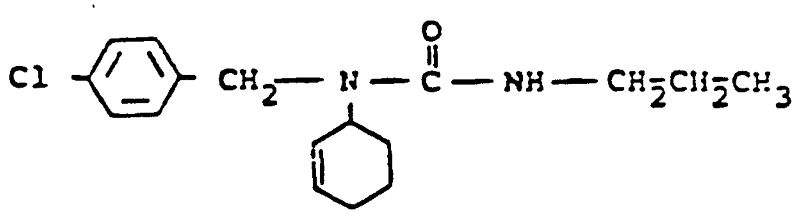

(Verbindung Nr. 1)

22 g N-4-Chlorobenzyl-2-cyclohexenylamin wurden in 400 ml Hexan gelöst, und unter Rühren und Eiskühlung der Lösung wurde eine Lösung von 9 g Propylisocyanat in 30 ml Hexan tropfenweise hinzugefügt. Nach der

Zugabe wurde die Temperatur allmählich erhöht, und das Rühren wurde bei 40°C etwa 3 h weiter fortgesetzt. Nach dem Abkühlen wurden die erhaltenen Kristalle unter Absaugen filtriert und aus einem Mischlösungsmittel aus Hexan-Ethylalkohol umkristallisiert, wonach 26,3 g des angestrebten Produkts, 1-(4-Chlorobenzyl)-1 -(2-cyclohexenyl)-3-propylharnstoff, erhalten wurden. Schmp. 80,0-83,0°C.

**Beispiel 3**

[Beispiel für die Synthese der Verbindung der Formel (I) gemäß der Erfindung]

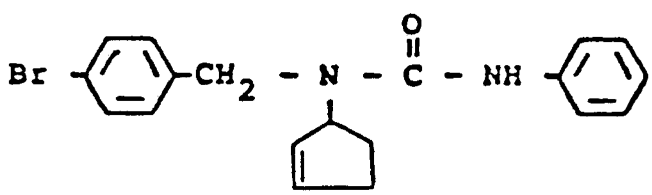

(Verbindung Nr. 2)

19 g Anilin wurden in 400 ml Toluol gelöst und unter Rühren und Eiskühlung der Lösung wurde eine Lösung von 31 q N-4-Bromobenzyl-N-(2-cyclopentenyl)carbamoylchlorid in 50 ml Toluol tropfenweise hinzugefügt. Nach der Zugabe wurde die Temperatur allmählich erhöht, und das Rühren wurde bei 70-80°C etwa 10 h weiter fortgesetzt. Nach dem Abkühlen wurde das kristallisierte Anilinhydrochlorid abfiltriert, und die Toluol-Schicht wurde nacheinander mit Wasser, einer 1-proz. wäßrigen Natriumcarbonat-Lösung, einer 1-proz. wäßrigen Salzsäure und Wasser gewaschen. Nach dem Waschen wurde die Toluol-Lösung über wasserfreiem Natriumsulfat getrocknet, das Toluol wurde abdestilliert, und der Rückstand wurde aus einem Mischlösungsmittel aus Hexan-Ethylalkohol umkristallisiert, wonach 26,7 g des angestrebten Produkts, 1-(4-Bromobenzyl)-1-(2-cyclopentenyl)-3-phenylharn-stoff, erhalten wurden. Schmp. 1 06,0-111,0°C.

Die Verbindungen der Formel (I) gemäß der vorliegenden Erfindung, die nach Verfahrensweisen ähnlich der in den Beispielen 2 und 3 beschriebenen hergestellt wurden, sind in der Tabelle 2 aufgeführt.

**Tabelle 2**

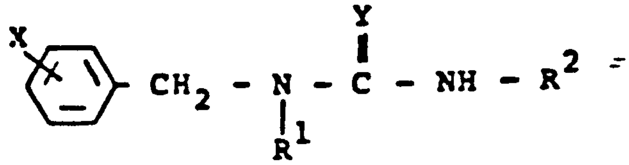

| Verbindung Nr. | X | $R^1$ | $R^2$ | Y | Physikal. Konstante: Schmp. |
|---|---|---|---|---|---|
| 3 | 4-Cl | 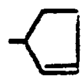 | $-CH_3$ | O | 82.0 - 88.0 |
| 4 | 4-Cl | 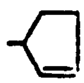 | $-C_2H_5$ | O | 88.5 - 90.5 |
| 5 | 4-Cl | 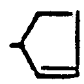 | $-C_3H_7$-n | O | 71.0 - 76.0 |
| 6 | 4-Cl | 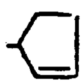 | $-C_4H_9$-n | O | 53.0 - 55.5 |
| 7 | 4-Cl | 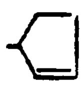 | 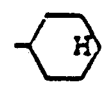 | O | 101.0 - 104.0 |
| 8 | 4-Cl | 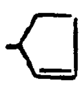 | 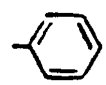 | O | 110.0 - 111.5 |
| 9 | 4-Cl | 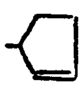 | 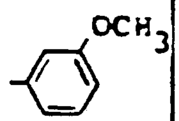 | O | 103.0 - 106.0 |
| 10 | 4-Br | 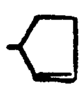 | $-CH_3$ | O | 74.0 - 76.0 |
| 11 | 4-Br | 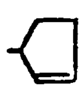 | $-C_2H_5$ | O | 83.0 - 86.0 |
| 12 | 4-Br | 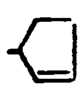 | $-C_3H_7$-iso | O | 63.0 - 68.0 |
| 13 | 4-$CH_3$ | 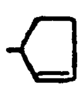 | $-C_2H_5$ | O | 91.0 - 93.0 |
| 14 | 4-$CH_3$ | 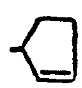 | $-C_3H_7$-n | O | 75.0 - 80.0 |
| 15 | 4-$CH_3$ | 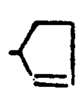 | 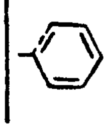 | O | 95.5 - 96.5 |

**Tabelle 2** - Fortsetzung

| Verbindung Nr. | X | $R^1$ | $R^2$ | Y | Physikal.Konstante:Schmp. |
|---|---|---|---|---|---|
| 16 | 4-Cl | 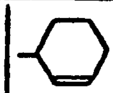 | $-CH_3$ | O | 105.0 - 106.0 |
| 17 | 4-Cl | 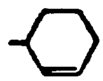 | $-C_2H_5$ | O | 96.0 - 98.0 |
| 18 | 4-Cl | 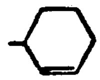 | $-C_4H_9$-n | O | 62.0 - 64.0 |
| 19 | 4-Cl | 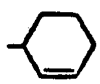 | 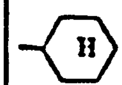 | O | 95.0 - 97.0 |
| 20 | 4-Cl | 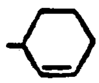 | 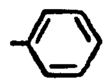 | O | 131.5 - 132.5 |
| 21 | 4-Cl | 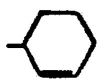 | 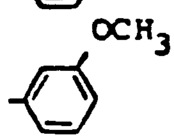 | O | 92.0 - 96.0 |
| 22 | 4-Br | 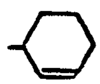 | $-C_3H_7$-n | O | 79.0 - 80.0 |
| 23 | 4-Br | 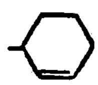 | 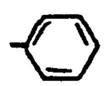 | O | 130.0 - 131.0 |
| 24 | $4-CH_3$ | 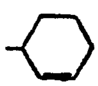 | $-C_2H_5$ | O | 68.0 - 71.0 |
| 25 | $4-CH_3$ | 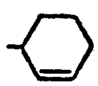 | $-C_3H_7$-n | O | 59.0 - 63.0 |
| 26 | $4-CH_3$ | 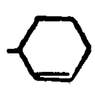 | 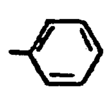 | O | 93.0 - 94.5 |
| 27 | $4-CH_3$ | 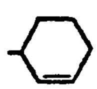 | 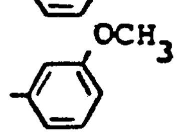 | O | 100.0 - 104.0 |
| 28 | 4-Cl | 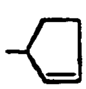 | $-C_2H_5$ | S | 98.5 - 100.0 |
| 29 | 4-Cl | 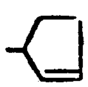 | 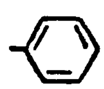 | S | 130.0 - 133.0 |
| 30 | 4-Br | 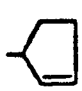 | $-CH_2-$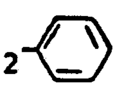 | S | 111.0 - 118.0 |

**Tabelle 2** - Fortsetzung

| Verbindung Nr. | X | $R^1$ | $R^2$ | Y | Physikal. Konstante: Schmp. |
|---|---|---|---|---|---|
| 31 | 4-Br | 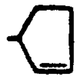 | 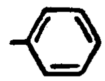 | S | 136.0 - 141.0 |
| 32 | $4-CH_3$ | 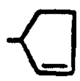 | $-C_4H_9-n$ | S | 52.0 - 54.0 |
| 33 | 4-Cl | 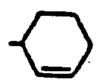 | $-C_2H_5$ | S | 96.0 - 97.5 |
| 34 | 4-Cl | 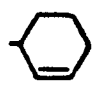 | 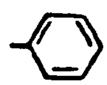 | S | 129.0 - 132.5 |
| 35 | $4-CH_3$ | 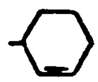 | $-C_2H_5$ | S | 87.5 - 88.5 |

Weiterhin wurden nach Arbeitsweisen ähnlich denjenigen in Beispiel 2 die nachstehenden Verbindungen der Formel (I) gemäß der vorliegenden Erfindung hergestellt:

1-(4-Chlorobenzyl)-1-(2-cyclopentenyl)-3-(3-hydroxyphenyl)harnstoff,
1-(4- Chlorobenzyl)-1-(2-cyclohexenyl)-3-(4-hydroxyphenyl)harnstoff,
1-(4-Chlorobenzyl)-1-(2-cyclohexenyl)-3-(4-methoxyphenyl)harnstoff,
1-(4-Chlorobenzyl)-1-(3-cyclopentenyl)-3-phenylharnstoff,
1-(4-Chlorobenzyl)-1-(3-cyclohexenyl)-3-propylharnstoff,
1-(4-Chlorobenzyl)-1-(2-cycloheptenyl)-3-phenylharnstoff.

Die Präparate der Verbindungen gemäß der vorliegenden Erfindung und die mit ihnen durchgeführten biologischen Tests werden mit Hilfe der folgenden Beispiele spezifisch erläutert. Die Bezugszahlen der Verbindungen entsprechen den vorstehenden.

**B. Formulierungsbeispiele**

**Beispiel 4:**

(Benetzbares Pulver)

15 Teile der Verbindung Nr. 1 der vorliegenden Erfindung, 80 Teile eines Gemisches (1:5) aus Diatomit-Pulver und Tonpulver, 2 Teile Natriumalkylbenzolsulfonat und 3 Teile eines Kondensats aus Natriumalkylnaphthalinsulfonat und Formalin wurden gemahlen und zu einem benetzbaren Pulver vermischt. Dieses wird mit Wasser verdünnt und auf pathogene Pilze und/oder die Orte, an denen sie wachsen und Pflanzenkrankheiten auftreten, durch Sprühen aufgebracht.

**Beispiel :5**

(Emulgierbares Konzentrat)

30 Teile der Verbindung Nr. 2 der vorliegenden Erfindung, 55 Teile Xylol, 8 Teile Polyoxyethylenalkylphenylether und 7 Teile Calciumalkylbenzolsulfonat wurden unter Rühren miteinander vermischt, wodurch ein emulgierbares Konzentrat hergestellt wurde. Dieses wird mit Wasser verdünnt und auf pathogene Pilze und/oder die Orte, an denen sie wachsen und Pflanzenkrankheiten auftreten, durch Sprühen aufgebracht.

**Beispiel :6**

(Stäubemittel)

2 Teile der Verbindung Nr. 3 der vorliegenden Erfindung und 98 Teile Tonpulver wurden gemahlen und gemischt, wodurch ein Stäubemittel hergestellt wird. Dieses wird auf pathogene Pilze und/oder die Orte, an denen sie wachsen und Pflanzenkrankheiten auftreten, aufgestäubt.

**Beispiel :7**

(Stäubemittel)

1,5 Teile der Verbindung Nr. 4 der vorliegenden Erfindung, 0,5 Teile Isopropylhydrogenphosphat (PAP) und 98 Teile Tonpulver wurden gemahlen und zusammengemischt, wodurch ein Stäubemittel hergestellt wird. Dieses wird auf pathogene Pilze und/oder die Orte, an denen sie wachsen und Pflanzenkrankheiten auftreten, aufgestäubt.

**Beispiel :8**

(Granulat)

25 Teile Wasser wurden zu einer Mischung aus 10 Teilen der Verbindung Nr. 5 der vorliegenden Erfindung, 30 Teilen Bentonit (Montmorillonit), 58 Teilen Talkum und 2 Teilen Ligninsulfonat zugesetzt, und das Gemisch wurde innig geknetet und unter Verwendung eines Extruder-Granulators zu Granulat mit einer Korngröße von 0,43 bis 2,0 mm (10 bis 40 mesh) pelletisiert, das dann bei 40°C bis 50°C getrocknet wurde, wodurch ein Granulat hergestellt wird. Dieses wird auf pathogene Pilze und/ oder die Orte, an denen sie wachsen und Pflanzenkrankheiten auftreten, durch Streuen aufgebracht.

**Beispiel :9**

(Granulat)

Ein Drehmischer wurde mit 95 Teilen Tonmineral-Teilchen mit einer Teilchengrößen-Verteilung im Bereich von 0,2 bis 2 mm beschickt, und unter Drehen des Mischers wurden 5 Teile der Verbindung Nr. 5 der vorliegenden Erfindung, gelöst in einem organischen Lösungsmittel auf die Tonmineral-Teilchen aufgesprüht, so daß es gleichmäßig absorbiert wurde, wodurch ein Granulat hergestellt wird. Dieses wird auf pathogene Pilze und/oder die Orte, an denen sie wachsen und Pflanzenkrankheiten auftreten, durch Streuen aufgebracht.

**Beispiel :10**

(Öl-Präparat)

0,5 Teile der Verbindung Nr. 7 der vorliegenden Erfindung und 99,5Teile Kerosin wurden unter Rühren vermischt, wodurch ein Öl-Präparat hergestellt wird. Dieses wird auf pathogene Pilze und/oder die Orte, an denen sie wachsen und Pflanzenkrankheiten auftreten, durch Sprühen aufgebracht.

## C. Verwendungsbeispiele

**Beispiel 11**

**Test der Wirksamkeit der Bekämpfung von Pellicularia sasakii durch Sprühen (Topf-Test):**

**Herstellung des Test-Präparates**

Wirkstoff: 50 Gew.-Teile;
Träger: 45 Gew.-Teile einer Mischung (1: 5) aus Diatomit und Kaolin;
Emulgator: 5 Gew.-Teile Polyoxyethylenalkylphenylether.

Der oben genannte Wirkstoff, der Träger und der Emulgator wurden in den angegebenen Mengen miteinander vermischt, wodurch ein benetzbares Pulver gebildet wurde, und eine vorher festgelegte Menge des benetzbaren Pulvers wurde mit Wasser verdünnt, wodurch eine Formulierung hergestellt wurde.

**Test-Verfahren**

Reis (Varietät: Kinmaze) wurde in Wagner-Töpfen (2 $dm^2$) im voll bewässerten Zustand kultiviert. Im frühen Stadium der Ahren-Bildung wurde die wie im Vorstehenden angegeben hergestellte Verdünnung des Test-Präparates in vorherbestimmter Konzentration in einer Menge von 100 ml auf jeweils 3 Töpfe aufgesprüht. Am Tage nach dem Sprühen wurden die Test-Reispflanzen am unteren Ende der Stengel mit Pellicularia sasakii beimpft, der 10 Tage in einem Gerste-Medium bis zur Bildung von Sklerotia kultiviert worden war, und zwecks Infizierung 10 Tage in einer feuchten Kammer gehalten, in der eine Temperatur von 28°C bis 30°C und eine relative Luftfeuchtigkeit von 95 % oder höher aufrechterhalten wurden. Danach wurden der Grad der Erkrankung und das Auftreten von Phytotoxizität untersucht. Die Prüfung erfolgte durch Messung des Grades der Ausdehnung der Schädigung von den beimpften Teilen der Wurzeln her, und der Grad der Schädigung wurde mit Hilfe des nachstehenden Standards ausgedrückt:

$$\text{Grad der Schädigung} = \frac{3n_3 + 2n_2 + n_1 + n_0}{3\,N} \times 100$$

Hierin bedeuten

N die Gesamtzahl der untersuchten Stengel, $n_0$ die Zahl der durch die Erkrankung nicht infizierten Stengel,

$n_1$ die Zahl der Stengel, die (von unten her) bis hinauf zu einer Schale des ersten Blattes befallen waren,

$n_2$ die Zahl der Stengel, die (von unten her) bis hinauf zu einer Schale des zweiten Blattes befallen waren, und

$n_3$ die Zahl der Stengel, die (von unten her) bis hinauf zu einer Schale des dritten Blattes oder darüber hinaus befallen waren.

Die Ergebnisse sind in der Tabelle 3 dargestellt.

In diesem Test zeigen z.B. die folgenden Verbindungen Nr. 1, 2, 5, 8, 7, 8, 9, 15, 18, 17, 18, 19, 20, 21, 22, 23, 28, 27, 29, 31, 33 und 34 gemäß der vorliegenden Erfindung im Vergleich zum Stand der Technik eine deutlich überlegene Aktivität.

**Tabelle 3**

| Verbindung[1] Nr. | Wirkstoff-Konzentration (ppm) | Grad der Schädigung % | Phyto-toxizität[4] |
|---|---|---|---|
| 1 | 50 | 0 | - |
| 2 | 50 | 0 | - |
| 5 | 50 | 0 | - |
| 6 | 250 | 0 | - |
| 7 | 250 | 0 | - |
| 8 | 50 | 0 | - |
| 9 | 250 | 0 | - |
| 15 | 50 | 0 | - |
| 16 | 250 | 0 | - |
| 17 | 50 | 0 | - |
| 18 | 50 | 0 | - |
| 19 | 50 | 0 | - |
| 20 | 50 | 0 | - |
| 21 | 50 | 0 | - |
| 22 | 50 | 0 | - |
| 23 | 50 | 0 | - |
| 26 | 50 | 0 | - |
| 27 | 50 | 0 | - |
| 29 | 50 | 0 | - |
| 31 | 50 | 0 | - |
| 33 | 250 | 0 | - |
| 34 | 50 | 0 | - |
| X-1 (Vergleichs-Verbindung) | 250 | 80,5 | - |
| X-2 (Vergleichs-Verbindung) | 250 | 80,0 | - |
| X-3 | 250 | 73,5 | - |

Anmerkungen zu Tabelle 3: 1) X-1 (Vergleichs-Verbindung)

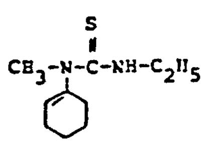

(Verbindung beschrieben in der US-PS 3 701 807)

X-2 (Vergleichs-Verbindung)

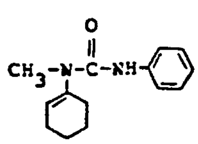

(Verbindung beschrieben in der US-PS 3 701 807)

X-3 (Vergleichs-Verbindung)

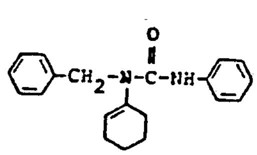

(Verbindung fällt in den Geltungsbereich der allgemeinen Formel, die beschrieben ist in der US-PS 3 701 807)

Polyxin:
Zink-Salz der polyoxin-Verbindung (2,2-proz. flüssiges Präparat von Polyoxin D-Zn).
Validamycin
A3-proz. flüssiges Präparat von Validamycin A.
2) Das Symbol "-" in der Spalte "Phytotoxizität" der Tabelle gibt an, daß keine Phytotoxizität auftrat.

**Beispiel 12**

**Test der Wirksamkeit der Bekämpfung von Rhizoctonis solani (im Gewächshaus):**

Dieses Beispiel zeigt die Ergebnisse des Tests der Bekämpfungswirkung gegen den im Boden lebenden pathogenen Pilz Rhizoctonia solani, der den Wurzelbrand der Keimlinge verschiedener Nutzpflanzen hervorruft, bei Bodenbehandlung.

**Herstellung der Test-Formulierungen**

3 Gew.-Teile der aktiven Verbindung und 97 Gew.-Teile Talkum wurden gemahlen und miteinander vermischt.

**Test-Verfahren**

Infizierter Boden wurde hergestellt durch Beimpfen eines durch Autoklavieren sterilisierter landwirtschaftlichen Ackerbodens (Tonlehm) mit Rhizoctonia solani, das 10 Tage in einem Weizenkleie-Medium kultiviert worden war, und das wie im Vorstehenden angegeben hergestellte Stäubemittel wurde in vorher festgelegter Konzentration in den Boden eingearbeitet vermischt, und die Mischung wurde zur Durchführung der Bodenbehandlung gut durchgerührt. Dieser chemikalienbehandelte Boden sowie unbehandelter Boden zu Vergleichszwecken wurden jeweils in Kunststoffbehälter mit einer Fläche von 27 x 18 $cm^2$ und einer Tiefe von 9 cm eingefüllt, so daß Saatbetten erhalten wurden. Dann wurden Samen von Gurken und von Auberginen, jeweils 50 Samenkörner pro Pflanzgefäß, eingesät, zur Herbeiführung der Keimung in einem Gewächshaus den üblichen Anzuchtbedingungen ausgesetzt, und die Anzahl der infizierten Sämlinge sowie die Phytotoxizität gegenüber der Keimung und dem Wachstum der Gemüsepflanzen wurden in vorher festgelegten Zeitabständen untersucht. Aufgrund der Testergebnisse am 25. Tage zeigten die Verbindungen Nr. 1. 2, 20, 23 und 25 in einer Wirkstoff-Konzentration von 25 ppm eine Bekämpfungswirkung von nahezu 100 % gegen den Wurzelbrand der Keimpflanzen von Gurken und Auberginen.

**Patentansprüche**

1. Substituierte Benzylcycloalkenylharnstoff-Derivate der Formel (I)

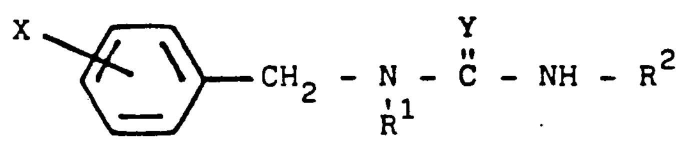

(I)

in der
X ein Halogen-Atom oder eine niedere Alkyl-Gruppe mit 1 bis 5 Kohlenstoff-Atomen bezeichnet,
Y ein Sauerstoff-Atom oder ein Schwefel-Atom bezeichnet,

$R^1$ eine Cycloalkenyl-Gruppe mit 5 bis 7 Kohlenstoff-Atomen bezeichnet und

$R^2$ eine niedere Alkyl-Gruppe mit 1 bis 6 Kohlenstoff-Atomen, eine Cycloalkyl-Gruppe mit 5 bis 8 Kohlenstoff-Atomen, eine Benzyl-Gruppe oder eine Phenyl-Gruppe bezeichnet, die gegebenenfalls 1 bis 5 fach gleich oder verschieden substituiert ist durch Hydroxyl oder eine Alkoxy-Gruppe mit 1 bis 4 Kohlenstoff-Atomen.

2. Substituierte Benzylcycloalkenylharnstoff-Derivate nach Anspruch 1, wobei in der Formel (I)

X Fluor, Chlor, Brom, Iod, Methyl, Ethyl, n-Propyl, iso-Propyl, n-Butyl, sec-Butyl, iso-Butyl oder tert-Butyl bezeichnet,

Y ein Sauerstoff-Atom oder ein Schwefel-Atom bezeichnet,

$R^1$ 2-Cyclopenten-1-yl, 3-Cyclopenten-1 -yl, 2-Cyclo-hexen-1 -yl, 3-Cyclohexen-1-yl, 2-Cyclohepten-1-yl, 3-Cyclohepten-1-yl, bezeichnet und

$R^2$ Methyl, Ethyl, n-Propyl, iso- Propyl, n-Butyl, sec-Butyl, iso-Butyl, tert-Butyl, Cyclopentyl, Cyclohexyl, Cycloheptyl, Cyclooctyl, Benzyl, Phenyl, das gegebenenfalls 1 oder 2 fach, gleich oder verschieden substituiert ist durch Hydroxyl, Methoxy, Ethoxy, n-Propoxy oder iso-Propoxy, bezeichnet.

3. Verfahren zur Herstellung substituierter Benzylcycloalkenylharnstoff-Derivate der Formel (I)

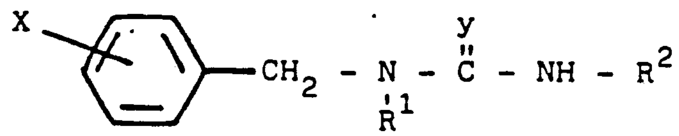

(I)

in der

X ein Halogen-Atom oder eine niedere Alkyl-Gruppe mit 1 bis 6 Kohlenstoff-Atomen bezeichnet,

Y ein Sauerstoff-Atom oder ein Schwefel-Atom bezeichnet,

$R^1$ eine Cycloalkenyl-Gruppe mit 5 bis 7 Kohlenstoff-Atomen bezeichnet und

$R^2$ eine niedere Alkyl-Gruppe mit 1 bis 8 Kohlenstoff-Atomen, eine Cycloalkyl-Gruppe mit 5 bis 8 Kohlenstoff-Atomen, eine Benzyl-Gruppe oder eine Phenyl-Gruppe bezeichnet, die gegebenenfalls substituiert ist durch 1 bis 5 gleiche oder verschiedene Substituenten, wie Hydroxyl oder eine Alkoxy-Gruppe mit 1 bis 4 Kohlenstoff-Atomen,

dadurch gekennzeichnet, daß

i) ein substituiertes Amin der Formel (II)

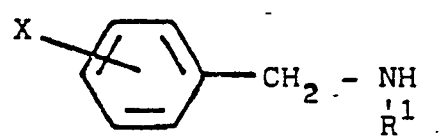

(II)

in der X und $R^1$ die im Vorstehenden angegebenen Bedeutungen haben, umgesetzt wird mit einem Isocyanat der Formel (III)

$R^2\text{-}N = C = Y$(III)

in der Y und $R^2$ die im Vorstehenden angegebenen Bedeutungen haben, gegebenenfalls in Gegenwart eines Verdünnungsmittels, oder

ii) ein Carbamoylhalogenid der Formel (IV)

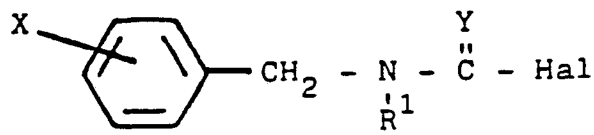

(IV)

in der X, Y und $R^1$ die im Vorstehenden angegebenen Bedeutungen haben und Hal ein Halogenatom ist, umgesetzt wird mit einem Amin der Formel (V)

$$H_2N - R^2 \qquad (V),$$

in der $R^2$ die im Vorstehenden angegebene Bedeutung hat, gegebenenfalls in Gegenwart eines Verdünnungsmittels sowie eines säurebindenden Mittels.

4. Mittel zur Pilzbekämpfung, dadurch gekennzeichnet, daß sie wenigstens ein substituiertes Benzylcycloalkenylharnstoff-Derivat der Formel (I) nach Anspruch 1 oder 3 enthalten.

5. Verwendung substituierter Benzylcycloalkenylharnstoff-Derivate der Formel (I) nach Anspruch 1 oder 3 zur Pilzbekämpfung.

6. Verfahren zur Pilzbekämpfung, dadurch gekennzeichnet, daß man substituierte Benzylcycloalkenylharnstoff-Derivate der Formel (I) nach Anspruch 1 oder 3 auf Pilze und/oder ihre Umwelt einwirken läßt.

7. Verfahren zur Herstellung von Mittelm zur Pilzbekämpfung, dadurch gekennzeichnet, daß substituierte Benzylcycloalkenylharnstoff-Derivate der Formel (I) nach Anspruch 1 oder 3 mit Streckmitteln und/oder oberflächenaktiven Mitteln vermischt werden.

8. Substituierte Benzyl-cycloalkenylamime der Formel (II)

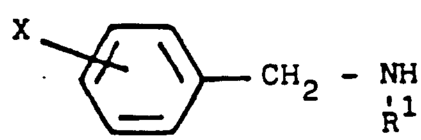

(II)

in der
X ein Halogen-Atom oder eine niedere Alkyl-Gruppe mit 1 bis 6 Kohlenstoffatomen bezeichnet und
$R^1$ eine Cycloalkenyl-Gruppe mit 5 bis 7 Kohlenstoffatomen bezeichnet.

9. Verfahren zur Herstellung subtituierter Benzylcycloalkenylamide der Formel (II)

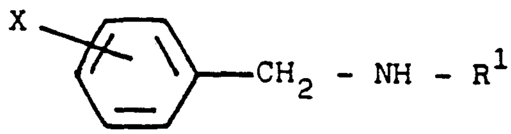

(II)

in der
X ein Halogen-Atom bezeichnet oder eine niedere Alkylgruppe mit 1 bis 6 Kohlenstoffatomen und
$R^1$ eine Cycloalkenylgruppe mit 5 bis 7 Kohlenstoffatomen bezeichnet,
dadurch gekennzeichnet, daß
a) ein substituiertes Benzylamin der Formel (VI)

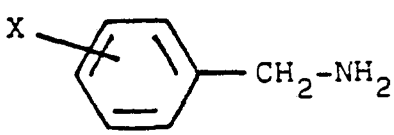

(VI)

in der X die oben angegebene Bedeutung hat, umgesetzt wird mit einer Verbindung der Formel
$R^1$ - Hal (VII)
in der $R^1$ die oben angegebene Bedeutung hat, und Hal ein Halogen-Atom ist, gegebenenfalls in Gegenwart eines Verdünnungsmittels und in Gegenwart eines säurebindenden Mittels bei Temperaturen zwischen -20° C und dem Siedepunkt der Reaktionsmischung oder
b) ein substituiertes Benzylhalogenid der Formel (VIII)

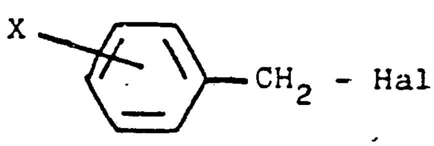

(VIII)

in der X und Hal die oben angegebenen Bedeutungen haben, umgesetzt wird mit einem Cycloalkenylamin der Formel (IX)
$R^1$-$NH_2$(IX),
in der $R^1$ die oben angegebene Bedeutung hat,
gegebenenfalls in Gegenwart eines Verdünnungsmittels und in Gegenwart eines säurebindenden Mittels bei Temperaturen zwischen -20° C und dem Siedepunkt der Reaktionsmischungen.

**Claims**

1. Substituted benzylcycloalkenyl urea derivatives of the formula (I)

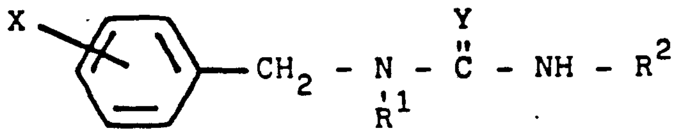

(I)

in which
X designates a halogen atom or a lower alkyl group with 1 to 8 carbon atoms,
Y designates an oxygen atom or a sulphur atom,
$R^1$ designates a cycloalkenyl group with 5 to 7 carbon atoms and
$R^2$ designates a lower alkyl group with 1 to 6 carbon atoms, a cycloalkyl group with 6 to 8 carbon atoms, a benzyl group or a phenyl group, which is optionally mono- to pentasubstituted by identical or different substituents selected from hydroxyl or an alkoxy group with 1 to 4 carbon atoms.

2. Substituted benzylcycloalkenyl urea derivatives according to Claim 1, wherein in the formula (I)
X designates fluorine, chlorine. bromine, iodine, methyl, ethyl, n-propyl, isopropyl, n-butyl, sec-butyl, isobutyl or tert-butyl,
Y designates an oxygen atom or a sulphur atom,
$R^1$ designates 2-cyclopenten-1 -yl, 3-cyclopenten-1 -yl, 2-cyclohexen-1 -yl, 3-cyclohexen-1 -yl, 2-cyclohepten-1-yl or 3-cyclohepten-1 -yl and
$R^2$ designates methyl, ethyl, n-propyl, isopropyl, n-butyl, sec-butyl, isobutyl, tert-butyl, cyclopentyl, cyclohexyl, cycloheptyl, cyclooctyl, benzyl or phenyl, which is optionally mono- or disubstituted by identical or different substituents selected from hydroxyl, methoxy, ethoxy. n-propoxy or isopropoxy.

3. Process for the preparation of substituted benzylcycloalkenyl urea derivatives of the formula (I)

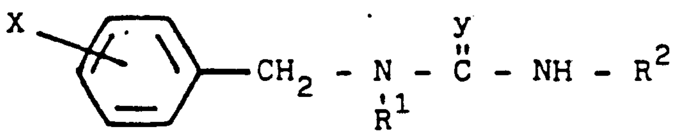

(I)

in which
X designates a halogen atom or a lower alkyl group with 1 to 6 carbon atoms,
Y designates an oxygen atom or a sulphur atom,
$R^1$ designates a cycloalkenyl group with 6 to 7 carbon atoms and
$R^2$ designates a lower alkyl group with 1 to 6 carbon atoms, a cycloalkyl group with 6 to 8 carbon atoms, a benzyl group or a phenyl group, which is optionally mono- to pentasubstituted by identical or different substituents such as hydroxyl or an alkoxy group with 1 to 4 carbon atoms, characterised in that
i) a substituted amine of the formula (II)

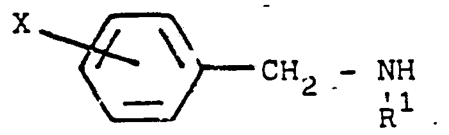

(II)

in which
X and $R^1$ have the abovementioned meanings, is reacted with an isocyanate of the formula (III)
$R^2$-N = C = Y(III)
in which
Y and $R^2$ have the abovementioned meanings, if appropriate in the presence of a diluent, or
ii) a carbamoyl halide of the formula (IV)

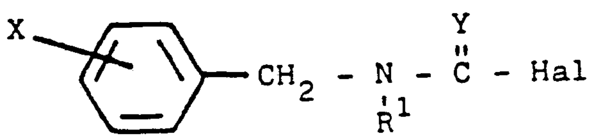

(IV)

in which
X, Y and $R^1$ have the abovementioned meanings and Hal is a halogen atom,
is reacted with an amine of the formula (V)
$H_2N$ - $R^2$(V),
in which
$R^2$ has the abovementioned meaning, if appropriate in the presence of a diluent and an acid-binding agent.

4. Agents for combating fungi, characterised in that they contain at least one substituted benzylcycloalkenyl urea derivative of the formula (I) according to Claim 1 or 3.

5. Use of substituted benzylcycloalkenyl urea derivatives of the formula (I) according to Claim 1 or 3 for combating fungi.

6. Method of combating fungi, characterised in that substituted benzylcycloalkenyl urea derivatives of the formula (I) according to Claim 1 or 3 are allowed to act on fungi and/or their environment.

7. Method of preparing agents for combating fungi, characterised in that substituted benzylcycloalkenyl urea derivatives of the formula (I) according to Claim 1 or 3 are mixed with extenders and/or surface-active agents.

8. Substituted benzylcycloalkenyl amines of the formula (II)

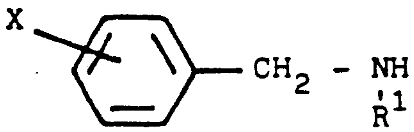

(II)

in which
X designates a halogen atom or a lower alkyl group with 1 to 6 carbon atoms and
$R^1$ designates a cycloalkenyl group with 6 to 7 carbon atoms.

9. Process for the preparation of substituted benzylcycloalkenyl amides of the formula (II)

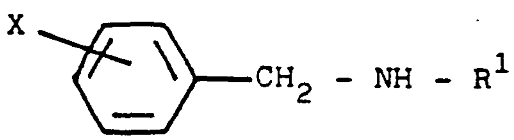

(II)

in which
X designates a halogen atom or a lower alkyl group with 1 to 6 carbon atoms and
$R^1$ designates a cycloalkenyl group with 6 to 7 carbon atoms, characterised in that
a) a substituted benzylamine of the formula (VI)

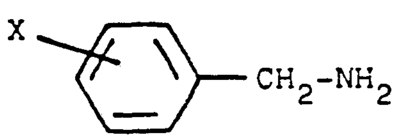

(VI)

in which
X has the abovementioned meaning, is reacted with a compound of the formula
$R^1$ - Hal (VII)
in which
$R^1$ has the abovementioned meaning and Hal is a halogen atom,
if appropriate in the presence of a diluent and in the presence of an acid-binding agent, at temperatures between -20° C and the boiling point of the reaction mixture or
b) a substituted benzyl halide of the formula (VIII)

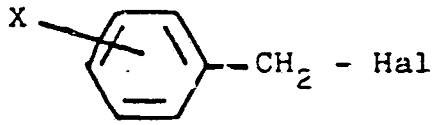

(VIII)

in which
X and Hal have the abovementioned meanings, is reacted with a cycloalkenylamine of the formula (IX)
$R^1 - NR_2$(IX)
in which
$R^1$ has the abovementioned meaning,
if appropriate in the presence of a diluent and in the presence of an acid-binding agent, at temperatures between -20°C and the boiling point of the reaction mixtures.

## Revendications

1. Dérivés substitués de benzyl-cycloalcényl-urées de formule (I):

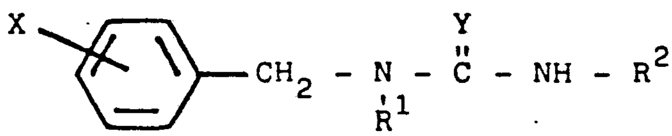

(I)

dans laquelle
X représente un atome d'halogène ou un groupe alkyle inférieur contenant 1 à 6 atomes de carbone,
Y représente un atome d'oxygène ou un atome de soufre,
$R^1$ représente un groupe cyclo-alcényle contenant 6 à 7 atomes de carbone, et,
$R^2$ représente un groupe alkyle inférieur contenant 1 à 6 atomes de carbone, un groupe cycloalkyle contenant 5 à 8 atomes de carbone, un groupe benzyle ou un groupe phényle qui est éventuellement substitué 1 à 5 fois de manière identique ou différente par un groupe hydroxy ou par un groupe alcoxy contenant 1 à 4 atomes de carbone.

2. Dérivés substitués de benzyl-cycloalcényl-urées selon la revendication 1 répondant à la formule (I) dans laquelle
X représente un atome de fluor, un atome de chlore, un atome de brome, un atome d'iode, un groupe méthyle, un groupe éthyle, un groupe n-propyle, un groupe isopropyle, un groupe n-butyle, un groupe sec-butyle,
Y représente un atome d'oxygène ou un atome de soufre,
$R^1$ représente un groupe 2-cyclopentén-1-yle, un groupe 3-cyclopentén-1-yle, un groupe 2-cyclohexén-1-yle, un groupe 3-cyclohexén-1-yle, un groupe 2-cyclo-heptén-1-yle ou un groupe 3-cycloheptén-1-yle, et
$R^2$ représente un groupe méthyle, un groupe éthyle, un groupe n-propyle, un groupe isopropyle, un groupe n-butyle, un groupe sec-butyle, un groupe isobutyle, un groupe tert-butyle, un groupe cyclopentyle, un groupe cyclohexyle, un groupe cycloheptyle, un groupe cyclo-octyle, un groupe benzyle ou un groupe phényle qui est éventuellement substitué 1 ou 2 fois de manière identique ou différente par un groupe hydroxy, par un groupe méthoxy, par un groupe ethoxy, par un groupe n-propoxy ou par un groupe isopropoxy.

3. Procédé de préparation de dérivés substitués de benzyl-cyclo-alcényl-urées de formule (I):

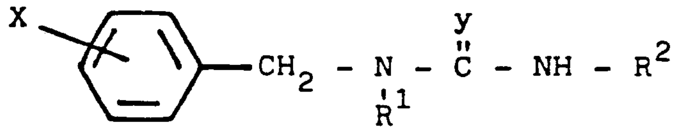

(I)

dans laquelle
X représente un atome d'halogène ou un groupe alkyle inférieur contenant 1 à 6 atomes de carbone,
Y représente un atome d'oxygène ou un atome de soufre,
$R^1$ représente un groupe cyclo-alcényle contenant 6 à 7 atomes de carbone, et
$R^2$ représente un groupe alkyle inférieur contenant 1 à 6 atomes de carbone, un groupe cyclo-alkyle contenant 5 à 8 atomes de carbone, un groupe benzyle ou un groupe phényle qui est éventuellement substitué par 1 à 5 substituants identiques ou differents tels qu-un groupe hydroxy ou un groupe alcoxy contenant 1 à 4 atomes de carbone,
caractérisé en ce que:
i) on fait réagir une amine substituée de formule (II):

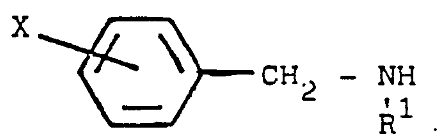

(II)

dans laquelle X et $R^1$ ont les significations indiquées ci-dessus, avec un isocyanate de formule (III):
$R^2\text{-}N = C = Y$(III)
dans laquelle Y et $R^2$ ont les significations indiquées ci-dessus, éventuellement en présence d'un diluant, ou
ii) on fait réagir un halogénure de carbamoyle de formule (IV):

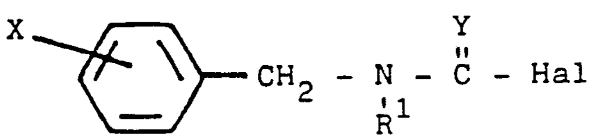

(IV)

dans laquelle X, Y et R ont les significations indiquées ci-dessus et Hal représente un atome d'halogène, avec une amine de formule (V):
$H_2N\text{-}R^2$ (V)
dans laquelle $R^2$ a la signification indiquée ci-dessus, éventuellement en présence d'un diluant, ainsi que d'un agent fixateur d'acide.

4. Agents destinés à combattre les champignons, caractérisés en ce qu'ils contiennent au moins un dérivé substitué de benzyl-cyclo-alcényl-urée de formule (I) selon la revendication 1 ou 3.

5. Utilisation de dérivés substitués de benzyl-cyclo-alcényl-urées de formule (I) selon la revendication 1 ou 3 en vue de combattre les champignons.

6. Procédé en vue de combattre les champignons, caractérisé en ce qu'on fait agir des dérivés substitués de benzyl-cyclo-alcényl-urées de formule (I) selon la revendication 1 ou 3 sur des champignons et/ou leur biotope.

7. Procédé de préparation d'agents en vue de combattre les champignons, caractérisé en ce qu'on mélange des dérivés substitués de benzyl-cyclo-alcénylurées de formule (I) selon la revendication 1 ou 3 avec des diluants et/ou des agents tensio-actifs.

8. Benzyl-cyclo-alcényl-amines substituées de formule (II):

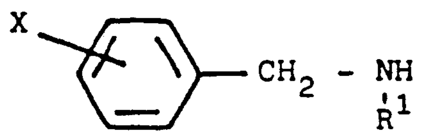

(II)

dans laquelle

X représente un atome d'halogène ou un groupe alkyle inférieur contenant 1 à 6 atomes de carbone, et

$R^1$ représente un groupe cyclo-alcényle contenant 6 à 7 atomes de carbone.

9. Procédé de préparation de benzyl-cycloalcényl-amines substituées de formule (II):

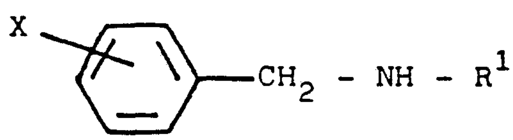

(II)

dans laquelle

X représente un atome d'halogène ou un groupe alkyle inférieur contenant 1 à 6 atomes de carbone, et $R^1$ représente un groupe cyclo-alcényle contenant 5 à 7 atomes de carbone,

caractérisé en ce que:

a) on fait réagir une benzylamine substituée de formule (VI):

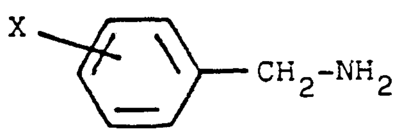

(VI)

dans laquelle X a la signification indiquée ci-dessus, avec un composé de formule:

$R^1$ - Hal(VII)

dans laquelle R a la signification indiquée ci-dessus et Hal représente un atome d'halogène, éventuellement en présence d'un diluant et en présence d'un agent fixateur d'acide, à des températures comprises entre -20°C

et le point d'ébullition du mélange réactionnel, ou

b) on fait réagir un halogénure de benzyle substitué de formule (VIII):

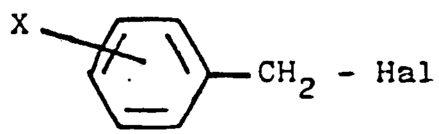

(VIII)

dans laquelle X et Hal ont les significations indiquées ci-dessus,

avec une cyclo-alcénylamine de formule (IX):

$R^1 - NH_2$(IX)

dans laquelle $R^1$ a la signification indiquée ci-dessus, éventuellement en présence d'un diluant et en présence d'un agent fixateur d'acide à des températures comprises entre -20°C et le point d'ébullition des mélanges réactionnels.